# EUROPEAN PATENT SPECIFICATION

(11) **EP 2 873 661 B1**
(45) Date of publication and mention of the grant of the patent: **07.10.2015**
(21) Application number: 13192912.7
(22) Date of filing: 14.11.2013
(51) Int. Cl.: C07D 319/06, B01J 23/00

(54) **Synthesis process of trimethylene carbonate from 1,3-propanediol and urea by heterogeneous catalysis**
Syntheseverfahren von Trimethylencarbonat aus 1,3-Propandiol und Harnstoff durch heterogene Katalyse
Procédé de synthèse de carbonate de triméthylène à partir de 1,3-propanediol et d'urée par catalyse hétérogène

(43) Date of publication of application: 20.05.2015
(73) Proprietor: ARKEMA FRANCE, 92700 Colombes (FR)
(72) Inventor: Dubois, Jean-Luc, 69390 Millery (FR); Aresta, Michele, 70124 Bari (IT); Dibenedetto, Angela, 70022 Altamura (Bari) (IT); Di Bitonto, Luigi, 76015 Trinitapoli (IT)

(56) References cited:
- EP-A1- 0 638 541
- EP-A1- 2 174 937
- JP-A- H07 330 756
- BHANAGE B M ET AL: "TRANSESTERIFICATION OF UREA AND ETHYLENE GLYCOL TO ETHYLENE CARBONATE AS AN IMPORTANT STEP FOR UREA BASED DIMETHYL CARBONATE SYNTHESIS", GREEN CHEMISTRY, ROYAL SOCIETY OF CHEMISTRY, CAMBRIDGE, GB, vol. 5, no. 4, 1 January 2003 (2003-01-01) , pages 429-432, XP008056370, ISSN: 1463-9262, DOI: 10.1039/B304182D
- LI Q ET AL: "Synthesis of cyclic carbonates from urea and diols over metal oxides", CATALYSIS TODAY, ELSEVIER, NL, vol. 115, no. 1-4, 30 June 2006 (2006-06-30), pages 111-116, XP027975915, ISSN: 0920-5861 [retrieved on 2006-06-30]

## Description

*The work leading to this invention has received funding from the European Union's Seventh Framework Programme (FP7*/*2007-2013) under grant agreement n°241718.*

The invention relates to the synthesis of trimethylene carbonate by reaction of alcoholysis of urea with 1,3-propanediol in liquid phase catalyzed by a mixed metal oxide comprising Zn and La.

Trimethylene carbonate hereunder shortly designated by "TMC" is a 6 membered-ring carbonate having the following developed formula

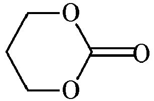

This carbonate is also named 1,3-dioxan-2-one according to the systematic classification.

The synthesis of trimethylene carbonate was described for years. Several methods have been proposed. For instance as illustrated by USP N° 6,506,909 the reaction of phosgene (COCl₂) and polyols (such as 1,3-propanediol) conducts to cyclic carbonates having a 5,6 or 7 atoms in accordance with the location of the hydroxyl functions of the polyol and 6 in the case of 1,3-propanediol.

An alternative way is the reaction of 1,3-propanediol with dialkylcarbonates. USP N° 5,212,321 describes such a reaction in the presence of Zn or Sn based compounds as catalysts. USP N° 6,054,596 describes a similar process using an alkaline or alkaline earth salt of a weak acid as catalyst.

Another method is the cycling-addition of CO₂ on a cyclic ether such as oxirane in the presence of Ph₄Sbl as catalyst as described by Akio Baba et al in an article " Cyclo-addition of oxetane and carbon dioxide catalyzed by tetraphenylstibonium iodide" in Tetrahedron Letters, Vol. 26, n° 10, pp 1323-1324 (1985).

The here-above methods present some severe drawbacks, phosgene being a deadly poison and cycling-addition using both expensive reagent and catalyst.

Recently a new chemical route has been proposed based on the reaction of 1,3-propanediol with urea according to the global reaction scheme as follows:

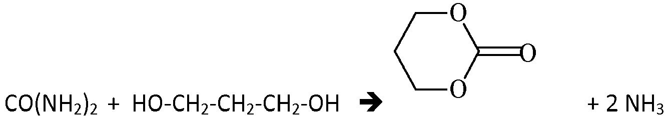

Such a process is described in the Mitsubishi Japanese patent N° 3800247 (Production of six-membered ring carbonate). In said patent an alkanediol having OH radicals in 1,3 or 2,4 positions (such as 1,3-propanediol for example) reacts with urea in the presence of a catalyst consisting of single metal such as Zn, Mg and Pb compounds at a temperature generally comprised between 120°C and 200°C under a low pressure in the range of 7-920 kPa. These catalysts are generally soluble at the reaction conditions and so cannot be easily recovered from the reaction medium.

In addition it is known that polytrimethylene carbonate (PTMC) can be used as raw material for producing TMC either by reactive distillation using a wiped film evaporator as described in US 7,005,496 or by enzymatic degradation as described by Shuichi Matsumara et al. "Lipase-Catalyzed Transformation of Poly(trimethylene carbonate) into Cyclic Carbonate" in Macromol. Rapid. Commun. 2001,22,pp 215-218*.*

The above mentioned global reaction scheme is in practice the result of two steps according to the following mechanism:
1) HO-CH₂-CH₂-CH₂-OH + NH₂-CO-NH₂ ↔ HO-CH₂-CH₂CH₂O-CO-NH₂ + NH₃,
   and

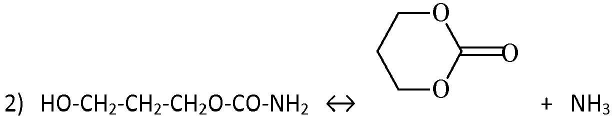

Both reactions are reversible so that in order to reach a high (total) conversion of reagents into TMC, the equilibrium of the reaction has to be shifted towards the right. The usual solution is the extraction of the gaseous NH₃ which is easy in applying to the reaction medium low pressure conditions. It is the way elected by Mitsubishi in the above-mentioned Japanese patent. The Mitsubishi patent recommends also to use an excess of 1,3-propanediol, 1,3-propanediol/urea ratio being comprised between 1 and 5 in specifying that a ratio lesser than 1 leads to a loss in selectivity and a ratio higher than 5 is economically unacceptable. These conditions shift the reaction to the right consuming most of the urea, but then remains the subsequent problem of separation of 1,3-propanediol and TMC.

The applicant has filed on October 8, 2008 a European patent application published as EP 2 174 937, dealing with said reaction polyol-urea conducting to cyclic polyol carbonates and more particularly to glycerol carbonates. The patent application was directed to the problem of the recovery of the cyclic polyol carbonate which was solved by the use in the reaction medium - or at least in a part of this medium - of a selective solvent for the cyclic polyol carbonate. Within the description of said application the applicant refers to several documents dedicated to the preparation of glycerol carbonate by reacting glycerol and urea.

These documents are EP 0 955 298 (ONIDOL), EP 1 156 042 (KAO) and in addition the article of J-W. Yoo and Z. Mouloungui «Catalytic carbonylation of glycerin by urea in presence of zinc mesoporous system for the synthesis of glycerol carbonate » in Studies in Surface Science and Catalysis 146 pages 757-760 Park et al (Editors) 2003. The operating conditions of the reaction described in these documents are very close:
Temperature: 90 < T°C < 220 ('298); 100 < T°C < 140 ('042); 130 < T°C < 150 (J-W. Yoo);
Pressure: 1 < P kPa < 20 ('298); 13.3 < P kPa < 101 ('042); 3 < P kPa < 4 (J-W. Yoo)
Catalysts:
   ('298) : metallic or organometallic sulfates, such as MnSO₄, ZnSO₄, MgSO₄, FeSO₄ or hydrate of paratoluene zinc sulfate
   ('042) : metal oxide (zinc oxide)
   J-W. Yoo : The article is dedicated to various catalyst systems using Zn as active element. In heterogeneous form zinc (ZnSO₄) is a mineral salt or associated with an acidic resin or on an aluminosilicate. In the homogeneous form, Zn is present as a p-toluene-sulphonate.

It appears that Zn is considered as the most effective metal catalyst for the reaction polyol-urea conducting to cyclic carbonates. However Zn solid catalysts such as ZnO which in a first phase are very active for forming cyclic carbonates from 1,3-diol and urea became less effective in the course of the reaction. The activity evolution of the catalyst is certainly due to a loss of solid ZnO which is partly solubilized in the liquid medium conducting to a lesser activity, and in addition catalyzing a side reaction concerning TMC which could be converted into linear carbonates. This major drawback leads in addition to a final reaction medium constituted by a mixture of chemical compounds, the separation of which being a particularly tricky issue especially in view of the final purification of TMC.

The problem to be solved by the invention is i) to improve both activity and selectivity, and to improve the stability of the Zn based catalyst in the course of reaction in order to increase the rate of reaction and the recovery of the catalyst at the end of the reaction run in view of a reuse, and consequently ii) to make easier the further step of separating the compounds of the reaction medium (product versus catalyst and other reactants) with an improved recovering rate in TMC.

The purpose of the invention is to provide a TMC synthesis process by heterogeneous catalysis using a new solid catalyst presenting better activity and stability and supplying an additional separation/purification step for extracting TMC from the reaction medium.

The invention is relating to a process for synthesizing trimethylene carbonate which comprises reacting 1,3-propanediol with urea in liquid phase, in the presence of a solid catalyst comprising a mixed Zn-La oxide, extracting produced NH₃, the resulting reaction medium being submitted to an additional step for separating the compounds constituting said reaction medium allowing to extract trimethylene carbonate from it.

The mixed Zn-La oxide catalyst may in addition contain other metals such as alkaline and alkaline earth metals, Scandium, Yttrium, Cadmium, Thallium, Lead, Antimony.

The reaction is preferably conducted at a temperature comprised between 90 °C and 200 °C, preferably at a temperature of 120°C to 170 °C and more preferably at about 130-155 °C.

The reaction is generally carried out under a pressure comprised between 0.5 kPa and 200 kPa, preferably between 1 kPa and 150 kPa and more preferably between 1 kPa and 30 kPa.

The solid mixed oxide catalyst used in the process of the invention is an intimate mixture of Zinc oxide (ZnO) and Lanthanum oxide (La₂O₃) leading, according to the molar ratio La/Zn, to a new mixed metal oxide comprising a solid solution of Lanthanum in Zinc oxide or to a spinel like structure when the molar ratio La/Zn is equal to 1. The molar ratio La/Zn is generally comprised between 0.01 and 1.5 and preferably between 0.1 and 1.

The solid catalysts can be easily separated from the reaction medium for instance by simple filtration and reused in the same medium (or another one) for further step of conversion of 1,3-propanediol with urea.

As compared with the pure ZnO catalyst, the mixed oxide catalyst of the invention presents a widely higher stability as shown by its recovery rate w/w from the reaction medium allowing to reuse the catalyst after very simple regenerating, in another runs. Considering the large quantities of catalyst to be used in the process, the easy recovery and regenerating is an appreciable advantage.

In addition the catalyst of the invention allows reaching yields in TMC higher than the yields obtained with the individual metal oxides ZnO or La₂O₃. The Zn-La oxides show a higher basicity than the parent oxide ZnO, with a much greater number of strong basic sites, a higher stability (lower release of Zn in solution), and a better catalytic activity with a high selectivity towards TMC.

The mixed metal oxide is obtained by any known usual mean for instance by simple mixing or more sophisticated techniques, the individual oxides being previously calcined at a temperature of 550°C for removing traces of moisture.

The mixing of the individual oxides is conducted by any conventional method such as precipitation, gelation and flocculation, hydrothermal synthesis, decantation-filtration-centrifugation and washing, followed by drying and/or calcination, and shaping (forming).

The grinding of the mixed metal oxides is conducted by any conventional method such as cyclonic crushing - when the solid is already a fine powder, or mixer-crushing, or ball-crushing.

It is possible to make use of "reactive grinding" a technique which consists in a mechanical size reduction of solid particles resulting simultaneously into a chemical reaction and a modification of the composition of the solid particles with in addition an agglomeration of said particles. Reactive grinding is generally conducted in using a mechanical energy generally comprised between 1 and 10000 Wh/kg and preferably between 10 and 1000 Wh/kg. Reactive grinding can be conducted in using any means known in the art such as centrifugal force, oscillation or a combination of both of them.

For example such a mixing and reactive grinding of the two individual oxides can be achieved by mean of High Energy Milling (HEM) for 1h at a speed of 700 rpm.

The solid mixed metal oxide catalyst will be constituted by shaped particles having a size generally comprised between 1 and 10 mm and made of aggregates of particles of a size between 1 and 20 nm and preferably between 5 and 12 nm.

In addition the specific surface area of the catalyst will be comprised between 1 and 30 m²/g and preferably between 1. 80 and 5 m²/g.

The catalyst is introduced in the reaction medium in an amount representing between 1 and 20 % weight of the reagents and preferably between 2 and 12 % weight.

The reaction is conducted with an initial excess of 1,3-propanediol in comparison with the stoichiometry of the reaction, the 1,3-propanediol in excess being the reactive solvent medium of the reaction. The starting molar ratios 1,3-propanediol/urea are comprised between 1/1 and 3/1 and preferably between 1/1 and 2/1.

The conversion of 1,3-propanediol into TMC depends on such molar ratio. The best yield is obtained when the PDO/Urea ratio is 1.5 that affords the best compromise for a high conversion of PDO and a high reaction selectivity defined by the molar ratio TMC/converted PDO or urea.

The selectivity depends on the nature of the catalyst. In fact, when ZnO is used the best yield (76%) is obtained after 9 h of reaction. For longer reaction times the yield in TMC decreases while the conversion of PDO increases, showing that new reactions implying TMC occur (most likely ring opening and oligomerization). Conversely, the La₂O₃-ZnO systems (La/Zn 0.5 and 1.0 molar ratio) produce the best yield after 12 h of reaction when the equilibrium is reached, and further heating does not produce any reduction of the concentration of TMC nor increase of the conversion of PDO. Similarly, the mixed oxide with a 0.1 molar ratio La/Zn requires a longer time to reach the same equilibrium position.

The second step separation/purification of TMC has been carried out following different approaches.

After recovering the solid catalyst from the reaction medium by filtration or centrifugation said used catalyst being regenerated by calcination, the resulting reaction mixture is submitted to a solvent extraction or to a distillation.

In one case the reaction mixture is dissolved in an organic such such as tetrahydrofuran (THF) and to the solution CH₂Cl₂ and water are added. The watery phase extracts the excess PDO and the residual unreacted monocarbamate HO-CH₂-CH₂-CH₂O-CO-NH₂ while the organic phase contains quite pure TMC. This system was able to afford 40-50% of 95-96% pure TMC as part of the formed TMC is trapped into an emulsion formed by PDO, CH₂Cl₂ and water, difficult to separate.

Alternatively, the reaction mixture is distilled *in vaccuo* preferably under a pressure of 3 mbar. The head of distillation collected contains PDO, the residual carbamate, and some TMC. The tail fraction was rich in TMC contaminated by some residual PDO. About 90% of the formed TMC is recovered.

In order to shift the equilibrium towards the formation of TMC, and convert residual PDO that also makes difficult the separation of TMC, short chain alcohol derived carbonates such as DMC (dimethyl carbonate), DEC (diethyl carbonate), DBC (dibutyl carbonate) and preferably DMC can be singularly added to the reaction medium, in a stoichiometric amount with respect to the excess PDO present at the equilibrium. A slight increment of formation ofTMC is observed. The remaining added carbonate reacts with PDO to afford linear carbonate (HO-CH₂-CH₂-CH₂O)₂CO determined by NMR.

This invention will be further understood in light of the following non-limiting examples which are given for illustration purposes only.

### EXAMPLES

### Preparation of the catalysts

The single metal oxides Al₂O₃, TiO₂, CeO₂, ZrO₂, Sc₂O₃, La₂O₃, ZnO and MgO are commercial ones which are in addition calcined for 3 h at 550°C for removing traces of moisture. CaO is obtained from calcining CaCO₃ for 3 h at 1100°C.

The mixed oxide La₂O₃ : ZnO (La:Zn ratio) is obtained by mixing and grinding of two individual oxides by mean of High Energy Milling for 1h at a speed of 700 rpm using a Pulverisette 7 apparatus (Fritsch).

### General operating conditions

In a typical run, 3.8 g of 1,3-propanediol (49.8 mmol) were reacted with urea (2 g, 33.2 mmol) [molar ratio 1,3-propanediol /urea=1.5] for 9-12 h (depending on the catalyst) at 150°C under 2 kPa pressure in presence of a catalyst at an amount representing 10% weight of the urea in a round bottomed flask connected to a vapor condenser (cooled at -10°C). The recovery of TMC was carried out following two routes, as described above.
A) At the end of the reaction (batch operation) the reaction mixture was cooled to room temperature and dissolved in 5 mL of THF. The solid catalyst was filtered off, 25 mL of CH₂Cl₂ and 25 mL of water were added to the THF solution in a separatory funnel and the two phases mixed vigorously. After phase separation, the watery phase contained the excess PDO, the residual unreacted monocarbamate HOCH₂CH₂CH₂O-C(O)NH₂ and some TMC, while the organic phase contained quite pure TMC with some traces of PDO. The extracted TMC was identified and quantified by GC-MS and ¹H and ¹³C NMR. It was isolated after solvent elimination *in vacuo* as 96% pure sample. In this way only 45 % of the formed TMC was recovered because a significant part was left in a emulsion formed by PDO-water and CH₂Cl₂, difficult to separate. However, this emulsion could be recycled in the following batch separation of the same kind, thereby reducing losses.
B) In a different attempt, the reaction mixture was distilled *in vacuo* under 3 mbar of pressure. The head of distillation collected at 61 °C contained the excess PDO and the residual monocarbamate, while the tails was formed of TMC (94 % pure) with residual monocarbamate and PDO.

### Example 1 (comparative): TMC synthesis from 1,3-propanediol (PDO) and urea with single metal oxides

The experiments have been conducted according to the general operating conditions in using as catalyst a set of single metal oxides. The Table 1 summarizes the obtained results. The yields in produced TMC are given in considering:
(1) the initial molar amount of urea (Yield TMC (1) = ratio mole TMC/mole urea initial *100) and
(2) the initial molar amount of 1,3-propanediol (Yield TMC (2) = ratio mole TMC/mole 1,3-PDO initial *100).

The recovery rate of catalyst considers the amount (in weight) of recovered catalyst compared with the initial one.

**TABLE 1**

| Exp. | Catalyst | Conversion 1,3-PDO (%) | Yield TMC (1) molar % | Yield TMC (2) molar % | Catalyst Recovery % weight |
|---|---|---|---|---|---|
| 1 | None | 36.8 | 4.3 | 2.9 | - |
| 2 | ZnO | 57.1 | 72.7 | 48.5 | (57.8)* |
| 3 | La₂O₃ | 49.7 | 46.8 | 31.2 | 95.0 |
| 4 | Sc₂O₃ | 56.4 | 15 | 9.9 | 90.0 |
| 5 | ZrO₂ | 51.5 | 10.4 | 6.9 | 86.2 |
| 6 | MgO | 41.6 | 9.8 | 6.5 | 92.8 |
| 7 | CeO₂ | 48.9 | 9.8 | 6.5 | 95.3 |
| 8 | CaO | 36.4 | 8.2 | 5.5 | 92.8 |
| 9 | TiO₂ | 37.9 | 8.1 | 5.4 | 93.1 |
| 10 | Al₂O₃ | 42.6 | 7.0 | 4.7 | 86.2 |

| | | | | | |
|---|---|---|---|---|---|
| * The recovered solid is not pure ZnO as demonstrated by its XRD. It is a mixture of ZnO (minority) and Zn(NCO)₂(NH₃)₂ (over 98%). | | | | | |

The data of experiments 2 and 3 show that ZnO has the highest conversion activity and La₂O₃ has a correct conversion and shows also a very high recovery rate and stability, much better than ZnO.

### Example 2: ZnO catalyst, influence of the reaction time.

The experiment 2 (pure ZnO as catalyst) was pursued for 15 h. instead of 7 h. and both the conversion rate in TMC and residual rate of PDO regularly controlled. The results obtained are summarized in Table 2.

**TABLE 2**

| Time (h.) | 1 | 3 | 5 | 7 | 9 | 11 | 13 | 15 |
|---|---|---|---|---|---|---|---|---|
| TMC Yield.* (%) | 16 | 32 | 55 | 72 | 76 | 74 | 71 | 69.5 |
| PDO Resid.** (%) | 65 | 49 | 48 | 45 | 40 | 33 | 28 | 23 |

| | | | | | | | | |
|---|---|---|---|---|---|---|---|---|
| TMC Yield.* means the ratio hereabove designed by Yield TMC (1) PDO Resid.** means the molar ratio PDO residual/ PDO initial | | | | | | | | |

These results show that the conversion into TMC increases for 9 h. to reach 76 % but slightly decreases thereafter while at the same time the PDO conversion is still increasing. Most likely, a side reaction takes place that converts TMC.

### Example 3: ZnO catalyst, influence of the initial molar ratio PDO/urea

In this example the experiments of the example 2 have been repeated two times in simply changing the charge in which an excess of PDO is added in order to reach the ratio PDO/urea of respectively 3/1 and 5/1. The results are summarized in Table 3.

**TABLE 3**

| Time (h.) | 1 | 3 | 5 | 7 | 9 | 11 | 13 | 15 |
|---|---|---|---|---|---|---|---|---|
| Yield TMC (1) (%) PDO/urea 1.5/1 | 16 | 32 | 55 | 72 | 76 | 74 | 71 | 69.5 |
| Yield TMC (1) (%) PDO/urea 3/1 | 10 | 22 | 37 | 47 | 50 | 48 | 47 | 45 |
| Yield TMC (1) (%) PDO/urea 5/1 | 4 | 12 | 19 | 22 | 23 | 22.5 | 22 | 21 |

With an excess of PDO the production of other products especially linear carbonates such as (HO-CH₂-CH₂-CH₂O)₂CO is induced explaining the lower yield into TMC

### Example 4 (according to the invention): Synthesis of TMC withLa:Zn oxides systems

For this example, 3 different catalysts have been prepared according to the method previously described.

Catalyst A is a mixture (about 0.6 g) of ZnO and La₂O₃ with a molar ratio "La:Zn" 0.1 prepared from 0.5011 g of ZnO and 0.1001 g of La₂O₃.

Catalyst B is a mixture (about 1.0 g) of ZnO and La₂O₃ with a molar ratio "La:Zn" 0.5 prepared from 0.5021 g of ZnO and 0.5013 g of La₂O₃.

Catalyst C is a mixture (about 1.5 g) of ZnO and La₂O₃ with a molar ratio "La:Zn" 1 prepared from 0.5021 g of ZnO and 1.0041 g of La₂O₃.

The measurement of the surface area of the catalysts according to BET method gives the following results: Catalyst A : 2.36 m²/g; Catalyst B : 1.90 m²/g and Catalyst C : 1.85 m²/g.

The surface area of the pure oxides prepared as catalysts is as follows : ZnO : 2.51 m²/g and La₂O_{3:}0.91 m²/g.

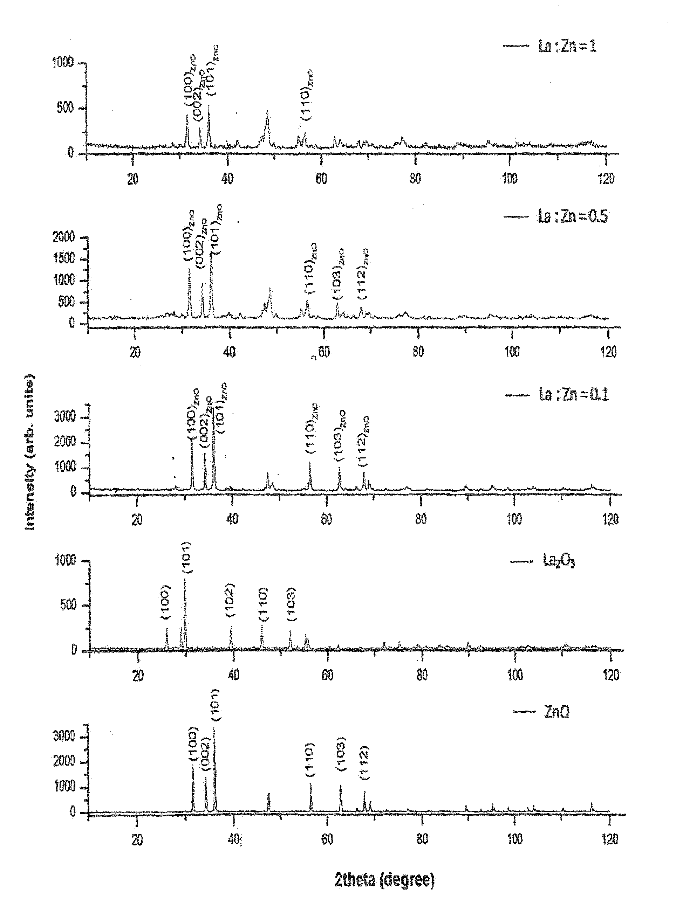

The PXRD (powder X-Ray diffraction) spectra of the five catalysts ZnO, La₂O₃ and three mixed oxides were conducted. Figure 1 shows the specific peaks corresponding to La₂O₃ are not present within the spectra of the three mixed oxides catalysts (A, B, C) demonstrating that a single phase (solid solution) based on ZnO (ZnO enriched with La₂O₃) is present.

The 1:1 (molar ratio) oxide mixture corresponds to a compound of formula ZnLa₂O₄, a "spinel-like compound". The profile of ZnLa₂O₄ is compatible with that of a compound of similar stoichiometry prepared by coprecipitation [S. Yan, S.O. Salley, K.Y.Simon Ng, Applied Catalysis A General, 353 (2009) 203-212]. The operating conditions are the general conditions but the reaction time is 12 h.

The results obtained are summarized in Table 4.

**TABLE 4**

| Exp. | Catalyst | Conversion 1,3-PDO (%) | Yield TMC (1) molar % | Yield TMC (2) molar % | Catalyst Recovery % w/w |
|---|---|---|---|---|---|
| 11 | A La:Zn 0.1 | 74.8 | 74.6 | 49.7 | 88 |
| 12 | B La:Zn 0.5 | 70.8 | 79.1 | 52.7 | 94 |
| 13 | C La:Zn 1 | 70.1 | 80.5 | 54.3 | 96.4 |
| 2 (ex 1) | ZnO | 57.1 | 72.7 | 48.5 | (57.8) |
| 3 (ex 1) | La₂O₃ | 49.7 | 46.8 | 31.2 | 95 |

Experiment 11 shows that with La:Zn 0.1 is obtained a surprising increase of the PDO conversion with respect to classical pure ZnO catalyst (exp. 2 of comparative example 1) and of course with respect to La₂O₃. (exp. 3 of comparative example 1).

Increasing the La content of the catalyst La:Zn leads to a slight increase of the catalytic activity (exp. 12 and 13). With increase of the conversion of PDO up to the equilibrium and a best yield of 80.5 % with a considerable increase of the recovery of the catalyst that reaches a best of 96.4 %.

The better catalytic activity of the mixed oxides catalysts as compared with ZnO catalyst activity is probably due to presence of dispersed lanthanum in zinc oxide leading to an increase of the content of strong basic sites as shown in Table 5 established by measurement of the CO2 desorption at 373-473°K (weak basic sites) and at 473-773°K (strong basic sites).

**TABLE 5**

| Entry | Catalysts | Volume of CO₂ (mL/m²) | % Basic sites | |
|---|---|---|---|---|
| | | | Weak | Strong |
| 1 | ZnO | 0.025 | 52 | 48 |
| 2 | La:Zn=0.1 | 0.442 | 17.6 | 82.4 |
| 3 | La:Zn=0.5 | 0.477 | 12.8 | 87.2 |
| 4 | La:Zn=1 | 0.511 | 10.9 | 89.1 |
| 5 | La₂O₃ | 0.249 | 22.9 | 77.1 |

Considering catalyst recovery it clearly appears that the catalyst stability is greatly improved for the mixed metal La:Zn oxides, the loss in solid metal catalyst compared with pure ZnO (Exp. 2) being divided by 2 or 3 depending on the La content.

It must also be emphasized that while the La:Zn oxides are recovered identical to the starting oxides, when ZnO is used, the solid recovered from the reaction medium is not anymore ZnO. Most of it (98 % w/w) is Zn(NCO)₂(NH₃)₂ formed upon reaction of ZnO with HNCO and NH3 formed from the decomposition of urea that occurs above 135 °C, according to the reactions reported below.

H₂NCONH₂ → HNCO + NH₃

ZnO + 2HNCO → "Zn(NCO)₂" + H₂O

"Zn(NCO)₂" + 2NH₃ → Zn(NCO)₂(NH₃)₂

This accounts for the superior activity of the La:Zn catalysts with respect to ZnO, due the higher basicity (that prevents the promotion of TMC conversion once formed), higher stability towards the conversion into other compounds, much better recoverability and reusability with the same catalytic efficiency.

## Claims

1. Process for synthesizing trimethylene carbonate which comprises reacting 1,3-propanediol with urea in liquid phase in the presence of a solid catalyst comprising a mixed Zn-La oxide, extracting produced NH₃, the resulting reaction medium being submitted to an additional step for separating the compounds constituting said reaction medium allowing to extract trimethylene carbonate from it.

2. Process according to claim 1 in which the temperature is comprised between 90 and 200 °C, preferably at a temperature of 120 to 170 °C and more preferably at about 130-155 °C.

3. Process according to claim 1 or 2 in which the pressure is comprised between 0.5 and 200 kPa, preferably between 1 and 150 kPa and more preferably between 1 and 30 kPa.

4. Process according to any claims 1 to 3 in which the mixed solid catalyst is an intimate mixture of Zn oxide (ZnO) and Lanthanum oxide (La₂O₃) with a molar ratio La:Zn comprised between 0.01 and 1.5 and preferably between 0.1 and 1.

5. Process according to any claims 1 to 4 in which the catalyst is introduced in the reaction medium in an amount representing between 1 and 20% weight of the reagents and preferably between 2 and 12 % weight.

6. Process according to any claims 1 to 5 in which the reaction is conducted with an initial excess of 1,3-propanediol, the starting molar ratios 1,3-propanediol/urea being comprised between 1/1 and 3/1 and preferably between 1/1 and 2/1.

7. Process according to any claims 1 to 6 in which the mixed oxide catalyst is constituted by shaped particles having a size generally comprised between 1 and 10 mm and made of aggregates of particles of a size between 1 and 20 nm and preferably between 5 and 12 nm.

8. Process according to any claims 1 to 7 in which the mixed oxide catalyst is prepared by reactive grinding generally conducted in using a mechanical energy comprised between 1 and 10000 Wh/kg and preferably between 10 and 1000 Wh/kg.

9. Process according to any claims 1 to 8 in which the mixed metal oxide catalyst comprises a solid solution of Lanthanum in Zinc oxide or a spinel like structure when the molar ratio La:Zn is equal to 1.

10. Process according to any claims 1 to 9 in which the additional step is conducted by distillation *in vaccuo* of the resulting reaction mixture after recovering the solid catalyst from the reaction medium.

## Patentansprüche

1. Verfahren zum Synthetisieren von Trimethylencarbonat, das Umsetzen von 1,3-Propandiol mit Harnstoff in flüssiger Phase in Gegenwart eines festen Katalysators, der ein gemischtes Zn-La-Oxid enthält, und Extrahieren des hergestellten NH₃ umfasst, wobei das entstehende Reaktionsmittel einem zusätzlichen Schritt zum Trennen der Verwindungen, aus denen das Reakti.onsmittel besteht, unterzogen wird, wodurch Trimethylencarbonat daraus extrahiert werden kann.

2. Verfahren nach Anspruch 1, bei dem die Temperatur zwischen 90 und 200 °C beträgt, vorzugsweise zwischen 120 und 170 °C und mehr bevorzugt zwischen etwa 130 und 155 °C.

3. Verfahren nach Anspruch 1 oder 2, bei dem der Druck zwischen 0,5 und 200 kPa beträgt, vorzugsweise zwischen 1 und 150 kPa und mehr bevorzugt zwischen 1 und 30 kPa.

4. Verfahren nach einem der Ansprüche 1 bis 3, bei dem der gemischte feste Katalysator eine innige Mischung von Zn-Oxid (ZnO) und Lanthanumoxid (La₂0₃) mit einem Molverhältnis La:Zn zwischen 0,01 und 1,5 und vorzugsweise zwischen 0,1 und 1 umfasst.

5. Verfahren nach einem der Ansprüche 1 bis 4, bei dem der Katalysator in das Reaktionsmittel in einem Anteil zwischen 1 und 20 Gew.-8 der Reagenzien und vorzugsweise zwischen 2 und 12 Gew.-% eingebracht wird.

6. Verfahren nach einem der Ansprüche 1 bis 5, bei dem die Reaktion mit einem anfänglichen Überschuss von 1,3-Propandiol erfolgt und die Ausgangsmolverhältnisse 1,3-Propandiol/Harnstoff zwischen 1/1 und 3/1 und vorzugsweise zwischen 1/1 und 2/1 liegen.

7. Verfahren nach einem der Ansprüche 1 bis 6, in dem der gemischte Oxidkatalysator aus geformten Teilchen einer Größe zwischen 1 und 10 mm und aus Aggregaten von Teilchen einer Größe zwischen 1 und 20 nm und vorzugsweise zwischen 5 und 12 nm besteht.

8. Verfahren nach einem der Ansprüche 1 bis 7, bei dem der gemischte Oxidkatalysator durch Reaktionsmahlen hergestellt wird, das im Allgemeinen unter Verwendung einer mechanischen Energie zwischen 1 und 10.000 Wh/kg und vorzugsweise zwischen 10 und 1000 Wh/kg erfolgt.

9. Verfahren nach einem der Ansprüche 1 bis 8, bei dem der gemischte Metalloxidkatalysator eine feste Lösung von Lanthan in Zinkoxid oder eine spinellartige Struktur aufweist, wenn das Molverhältnis La:Zn gleich 1 ist.

10. Verfahren nach einem der Ansprüche 1 bis 9, bei dem der zusätzliche Schritt durch Destillation unter Vakuum der hergestellten Reaktionsmischung erfolgt, nachdem der feste Katalysator aus dem Reaktionsmittel rückgewonnen wurde.

## Revendications

1. Procédé pour synthétiser du carbonate de triméthylène qui comprend la réaction de 1,3-propanediol avec de l'urée en phase liquide en présence d'un catalyseur solide comprenant un oxyde mixte de Zn-La, l'extraction du NH₃ produit, le mélange réactionnel résultant étant soumis à une étape supplémentaire pour séparer les composés constituant ledit mélange réactionnel, ce qui permet d'en extraire le carbonate de triméthylène.

2. Procédé selon la revendication 1, dans lequel la température est comprise entre 90 et 200°C, de préférence à une température de 120 à 170°C et plus préférablement à environ 130-155°C.

3. Procédé selon la revendication 1 ou 2, dans lequel la pression est comprise entre 0,5 et 200 kPa, de préférence entre 1 et 150 kPa et plus préférablement entre 1 et 30 kPa.

4. Procédé selon l'une quelconque des revendications 1 à 3, dans lequel le catalyseur solide mixte est un mélange intime d'oxyde de Zn (ZnO) et d'oxyde de lanthane (La₂O₃), le rapport molaire La:Zn étant compris entre 0,01 et 1,5 et de préférence entre 0,1 et 1.

5. Procédé selon l'une quelconque des revendications 1 à 4, dans lequel le catalyseur est introduit dans le milieu réactionnel en une quantité représentant entre 1 et 20% en poids des réactifs et de préférence entre 2 et 12 % en poids.

6. Procédé selon l'une quelconque des revendications 1 à 5, dans lequel la réaction est réalisée avec un excès initial de 1,3-propanediol, les rapports molaires initiaux 1,3-propanediol/urée étant compris entre 1/1 et 3/1 et de préférence entre 1/1 et 2/1.

7. Procédé selon l'une quelconque des revendications 1 à 6, dans lequel le catalyseur oxyde mixte est constitué de particules façonnées présentant une grosseur généralement comprise entre 1 et 10 mm et constituées d'agrégats de particules d'une grosseur entre 1 et 20 nm et de préférence entre 5 et 12 nm.

8. Procédé selon l'une quelconque des revendications 1 à 7, dans lequel le catalyseur oxyde mixte est préparé par broyage réactif généralement réalisé à l'aide d'une énergie mécanique comprise entre 1 et 10.000 Wh/kg et de préférence entre 10 et 1000 Wh/kg.

9. Procédé selon l'une quelconque des revendications 1 à 8, dans lequel le catalyseur oxyde métallique mixte comprend une solution solide de lanthane dans de l'oxyde de zinc ou une structure de type spinelle lorsque le rapport molaire La:Zn vaut 1.

10. Procédé selon l'une quelconque des revendications 1 à 9, dans lequel l'étape supplémentaire est réalisée par distillation sous vide du mélange réactionnel résultant après la récupération du catalyseur solide du milieu réactionnel.
